# EUROPEAN PATENT SPECIFICATION

(11) **EP 1 409 573 B1**
(45) Date of publication and mention of the grant of the patent: **12.03.2008**
(21) Application number: 02753789.3
(22) Date of filing: 21.03.2002
(51) Int. Cl.: C08J 9/00, C07C 19/08, C09K 3/30, C08J 9/14

(54) **MIXTURES CONTAINING 1,1,1,3,3-PENTAFLUOROPROPANE AND 1,1,1,3,3-PENTAFLUOROBUTANE**
GEMISCHE, DIE 1,1,1,3,3-PENTAFLUORPROPAN UND 1,1,1,3,3-PENTAFLUORBUTAN ENTHALTEN
MELANGES CONTENANT DU 1,1,1,3,3-PENTAFLUOROPROPANE ET DU 1,1,1,3,3-PENTAFLUOROBUTANE

(30) Priority: 21.03.2001 US 813494
(43) Date of publication of application: 21.04.2004
(60) Divisional application: 07102736.1
(73) Proprietor: Honeywell International, Inc., Morristown, New Jersey 07962 (US)
(72) Inventor: BOGDAN, Mary, Charlotte, West Seneca, NY 14206 (US); WILLIAMS, David, John, East Amherst, NY 14051 (US); RIEGAL, Ronald, Buffalo, NY 14210 (US); BEMENT, Leslie, Bruce, Buffalo, NY 14210 (US)
(74) Representative: Hucker, Charlotte Jane
(86) International application number: PCT/US2002/008570
(87) International publication number: WO 2002/076176

(56) References cited:
- EP-A- 0 381 986
- WO-A-01/44352
- WO-A-96/14354
- WO-A-98/27145
- WO-A-99/61519
- US-A- 5 574 192
- US-A- 5 837 743
- US-B1- 6 303 667
- DATABASE CAPLUS CHEMICAL ABSTRACTS SERVICE, COLUMBUS, OHIO, US; XP002292274 retrieved from STN Database accession no. 2002:538227 & JP 2002 201251 A (NIPPON PAFUTEMU KK) 19 July 2002 (2002-07-19)

## Description

### Field of the Invention

The invention relates to mixtures of 1,1,1,3,3-pentafluoropropane and 1,1,1,3,3-pentafluorobutane. More particularly the invention relates to a method of dissolving a contaminant or removing a contaminant from the surface of a substrate using such a mixture.

### Background

1,1,1,3,3-pentafluoropropane (HFC-245fa) and 1,1,1,3,3-pentafluorobutane (HFC-36Smfc) are known blowing agents for the production of foams. See U.S. Patent Nos. 5,496,866; 5,574,192; 5,917,098; and 6,080,799.

WO-A-98/27145 discloses the use of hydrofluorocarbons (HFCs) as agents for the production of foams and, in particular, discloses blowing agent compositions containing greater than 50 weight % HFC-365mfc and less than 50 weight % HFC-245fa for this purpose. Brief mention is also made of the possibility of using such compositions as cleaning agents.

EP-A-03 81986 is also directed to the use of HFCs as blowing agents for the production of foams. Brief mention is also made that the HFCs disclosed in this document may be used as degreasing or cleaning agents in the electronics industry.

### Summary

The invention provides a method of dissolving a contaminant or removing a contaminant from the surface of a substrate, which comprises the step of contacting the substrate with a composition comprising, consisting essentially of or consisting of 1,1,1,3,3-pentafluoropropane (HFC-245fa) and 1,1,1,3,3-pentafluorobutane (HFC-365mfc), in from 51 to 99 weight percent 1,1,1,3,3-pentafluoropropane and from 1 to 49 weight percent 1,1,1,3,3-pentafluorobutane. More preferably, the compositions comprise from 75 to 99 weight percent 1,1,1,3,3-pentafluoropropane and from 1 to 25 weight percent 1,1,1,3,3-pentafluorobutane.

The method of dissolving a contaminant or removing a contaminant from the surface of a substrate according to the invention comprises, consists essentially of, or consists of the step of contacting the substrate with the above-described compositions.

## Claims

1. A method of dissolving a contaminant or removing a contaminant from the surface of a substrate that comprises the step of contacting the substrate with a composition comprising from 51 to 99 weight percent 1,1,1,3,3-pentafluoropropane and from 1 to 49 weight percent 1,1,1,3,3-pentafluorobutane.

2. A method according to claim 1, wherein the composition comprises from 75 to 99 weight percent 1,1,1,3,3-pentafluoropropane and from 1 to 25 weight percent 1,1,1,3,3-pentafluorobutane.

## Patentansprüche

1. Verfahren zum Ablösen einer Verunreinigung oder Abtragen einer Verunreinigung von der Oberfläche eines Substrats, bei dem man das Substrat mit einer Zusammensetzung, die 51 bis 99 Gewichtsprozent 1,1,1,3,3-Pentafluorpropan und 1 bis 49 Gewichtsprozent 1,1,1,3,3-Pentafluorbutan enthält, in Berührung bringt.

2. Verfahren nach Anspruch 1, bei dem die Zusammensetzung 75 bis 99 Gewichtsprozent 1,1,1,3,3-Pentafluorpropan und 1 bis 25 Gewichtsprozent 1,1,1,3,3-Pentafluorbutan enthält.

## Revendications

1. Procédé permettant de dissoudre un contaminant ou d'éliminer un contaminant de la surface d'un substrat, qui comprend l'étape consistant à mettre en contact le substrat avec une composition comprenant de 51 à 99 pour cent en poids de 1,1,1,3,3-pentafluoropropane et de 1 à 49 pour cent en poids de 1,1,1,3,3-pentafluorobutane.

2. Procédé selon la revendication 1, dans lequel la composition comprend de 75 à 99 pour cent en poids de 1,1,1,3,3-pentafluoropropane et de 1 à 25 pour cent en poids de 1,1,1,3,3-pentafluorobutane.
